(19) Europäisches Patentamt
European Patent Office
Office européen des brevets

(11) Veröffentlichungsnummer: **0 233 843**
**A2**

(12) **EUROPÄISCHE PATENTANMELDUNG**

(21) Anmeldenummer: 87810089.0

(22) Anmeldetag: 16.02.87

(51) Int. Cl.³: C 08 G 59/14
C 08 G 59/06, H 01 L 23/30

(30) Priorität: 20.02.86 CH 675/86

(43) Veröffentlichungstag der Anmeldung:
26.08.87 Patentblatt 87/35

(84) Benannte Vertragsstaaten:
CH DE ES FR GB IT LI NL

(71) Anmelder: CIBA-GEIGY AG
Klybeckstrasse 141
CH-4002 Basel(CH)

(72) Erfinder: Pallie, Kemal Dean, Dr.
Outre-Vieze
CH-1871 Choêx(CH)

(72) Erfinder: Dessauges, Gérald, Dr.
Ruelle du Chêne 1
CH-1820 Montreux(CH)

# (54) Verfahren zur Verminderung des Chlorgehaltes in Glycidylverbindungen.

(57) Zur Verminderung des Chlorgehaltes in Glycidylverbindungen, deren Glycidylgruppen an O-, N- und/oder S-Atome gebunden sind, werden diese in einem halogenfreien, aprotischen organischen Lösungsmittel gelöst mit einem Ammonium-bicarbonat oder -alkylcarbonat umgesetzt, und das Produkt wird danach aus dem Reaktionsgemisch isoliert, wobei das Ammoniumsalz eine Verbindung der Formeln I oder II

$$\begin{array}{c} R^1 \\ | \\ R^4\text{–}N^+\text{–}R^2 HCO_3^- \\ | \\ R^3 \end{array} \quad \text{I} \qquad \begin{array}{c} R^1 \\ | \\ R^4\text{–}N^+\text{–}R^{2-}OCOOR \\ | \\ R^3 \end{array} \quad \text{II}$$

ist, worin $R^1$ bis $R^4$ unabhängig voneinander $C_1$-$C_8$-Alkyl, Phenyl, Benzyl, $C_1$-$C_8$-Alkoxy oder Phenoxy und R $C_1$-$C_4$-Alkyl bedeuten.

Wegen ihres geringen Chlorgehaltes eignen sich die so erhaltenen Glycidylverbindungen insbesondere als Umhüllungsharze für elektronische Komponenten und als Klebstoffe bzw. Schutzüberzüge bei der Befestigung elektronischer Komponenten auf Leiterplatten ("globe top", "conformer coating").

EP 0 233 843 A2

Croydon Printing Company Ltd.

CIBA-GEIGY AG 3-15763/-

Basel (Schweiz)

Verfahren zur Verminderung des Chlorgehaltes in Glycidylverbindungen

Die Erfindung betrifft ein Verfahren zur Verminderung des Chlorgehaltes in Glycidylverbindungen durch Reaktion mit Bicarbonaten oder Alkylcarbonaten, die nach diesem Verfahren erhaltenen Glycidylverbindungen sowie deren Verwendung.

Bekanntlich sind die mittels Epichlorhydrin hergestellten Glycidylverbindungen, insbesondere die technisch hergestellten, stets mit Chlor verunreinigt, das in der Glycidylverbindung als ionisches Chlor, als hydrolysierbares (α,β-Chlorhydrin) und/oder als nichthydrolysierbares Chlor (α,γ-Chlorhydrin) vorliegt.

An Epoxidharze, insbesondere solche, die zur Beschichtung von Metallen oder zur Herstellung von elektrischen und elektronischen Bauteilen Verwendung finden, werden laufend höhere Ansprüche bezüglich Reinheit gestellt, um den Korrosionseinfluss des Restchlorgehaltes auf Substrate, insbesondere Kontaktmetalle, zu vermindern.

Es sind bereits Methoden zur Verminderung des Restchlorgehaltes in Epoxidharzen bekannt geworden. Die DD-Patentschrift 218 375 beschreibt z.B. ein Verfahren zur Dehydrohalogenierung (Nachverseifung) von Epoxidharzen zur Erzielung von Endprodukten mit einem Gehalt an hydrolysierbarem Chlor von unter 0,1 Gew. %. Dazu wird das Epoxidharz in einem wässrig-organischen Medium in Gegenwart eines Puffersystems und eines Katalysators auf Basis von Oniumverbindungen mit einem Alkalihydroxid dehydrohalogeniert. Aus der JP-Offenlegungsschrift 85/54 376 ist ein Verfahren zur Verminderung des Gehaltes an hydrolysierbarem Chlor in Epoxidharzen durch Behandlung mit stark basischen Anionentauscherharzen bekannt. Die JP-Offenlegungsschrift 83/173 116 offenbart eine Herabsetzung des Restchlorgehaltes in Epoxidharzen mittels Silbersalzen organischer Säuren. Abgesehen davon, dass es sich hierbei um ein kostspieliges Verfahren handelt, haben eigene Versuche ergeben, dass der nach diesem Verfahren erzielte Effekt gering ist.

Aus dem Artikel von C. Venturello und R. D'Aloisio in Synthesis, 33 (1985) ist ferner bekannt, dass 1,2-Halohydrine durch eine Reaktion mit quaternären Ammonium-bicarbonaten zu 1,2-Alkandiylcarbonaten umgesetzt werden können, wobei als Nebenprodukte das entsprechende quaternäre Ammonium-halogenid und Wasser entstehen.

Es wurde nun gefunden, dass man durch Reaktion mit bestimmten Bicarbonaten oder Alkylcarbonaten den Restchlorgehalt in Glycidylverbindungen verringern kann, ohne dass die Epoxidgruppe dabei wesentlich angegriffen wird.

Gegenstand der vorliegenden Erfindung ist somit ein neues Verfahren zur Verminderung des Chlorgehaltes in Glycidylverbindungen, deren Glycidylgruppen an O-, N- und/oder S-Atome gebunden sind, indem man die in einem halogenfreien aprotischen organischen Lösungsmittel gelöste Glycidylverbindung mit einem Ammonium-bicarbonat oder -alkylcarbonat reagieren lässt und danach das Produkt aus der Reaktionslösung isoliert, dadurch gekennzeichnet, dass das Ammoniumsalz eine Verbindung der Formeln I oder II

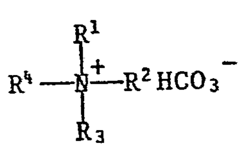

I

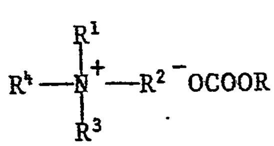

II

ist, worin $R^1$ bis $R^4$ unabhängig voneinander $C_1$-$C_8$-Alkyl, Phenyl, Benzyl, $C_1$-$C_8$-Alkoxy oder Phenoxy und R $C_1$-$C_4$-Alkyl bedeuten.

Das erfindungsgemässe Verfahren wird vorzugsweise unter einer $CO_2$- oder $N_2$-Atmosphäre durchgeführt.

Die in der Technik am meisten verwendeten Glycidylgruppen enthaltenden Epoxidharze weisen einen Epoxidgehalt von zwischen 2 und 6 Epoxidäquivalenten/kg auf. Es werden immer mehr Epoxidharze mit einem möglichst hohen Epoxidgehalt (mehr als 4 Aequivalenten/kg), der eine dichtere Vernetzung der ausgehärteten Harze ermöglicht, verlangt. Im Vergleich zum Epoxidgehalt ist der Chlorgehalt dieser Glycidylverbindungen viel tiefer und beträgt bei den grosstechnisch hergestellten Epoxidharzen meistens weniger als 1 Gew. % (0,28 Aequivalente/kg) und oft sogar weniger als 0,1 Gew. % (0,03 Aequivalente/kg). Bei einem so viel grösseren Gehalt an Epoxidgruppen als an Chloratomen (ein Ueberschuss von zwischen 7 bis 200 mal) bedarf es einer äusserst selektiven Reaktion, um die unerwünschten Halogenatome aus dem Harz zu entfernen, ohne dass dabei eine wesentliche Umsetzung an den Epoxidgruppen und eine Verminderung des Epoxidgehaltes eintreten. Bei dem erfindungsgemässen Verfahren handelt es sich um eine derartige selektive Reaktion, durch die der Gehalt an hydrolysierbarem sowie an nichthydrolysierbarem Chlor der Glycidylverbindung wesentlich vermindert wird, ohne dass der Epoxidgehalt beeinträchtigt wird. Dies ist um so überraschender, als es zum Beispiel aus der US-Patentschrift 2,873,282 oder aus der DE-Offenlegungsschrift 2 611 087 bekannt ist, dass Epoxide in Anwesenheit von Basen mit $CO_2$ reagieren können.

Für das erfindungsgemässe Verfahren können Glycidylverbindungen eingesetzt werden, die direkt an Sauerstoff-, Stickstoff- und/oder Schwefelatome gebundene Glycidylgruppen enthalten. Als Beispiele solcher Harze seien Polyglycidylester genannt, die man durch Umsetzung einer zwei oder mehr Carbonsäuregruppen pro Molekül enthaltenden Verbindung mit Epichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Pimelinsäure, Korksäure, Azelainsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, und von aromatischen Polycarbonsäuren, wie Phthalsäure, Isophthalsäure und Terephthalsäure, ableiten.

Weitere Beispiele sind Polyglycidyl- und Poly(ß-methylglycidyl)-ether, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Ether lassen sich mit Epichlorhydrin beispielsweise aus acyclischen Alkoholen, wie Ethylenglykol, Diethylenglykol, höheren Poly-(oxyethylen)-glykolen, Propan-1,2-diol, Poly-(oxy-propylen)-glykolen, Propan-1,3-diol, Poly-(oxytetramethylen)-glykolen, Pentan-1,5-diol, Hexan-2,4,6-triol, Glycerin, 1,1,1-Trimethylolpropan, Pentaerythrit und Sorbit, aus cycloaliphatischen Alkoholen, wie 1,3- und 1,4-Cyclohexandiol, Bis-(4-hydroxycyclohexyl)-methan, 2,2-Bis-(4-hydroxycyclohexyl)-propan und 1,1-Bis-(hydroxymethyl)-cyclohexen-3, und aus Alkoholen mit aromatischen Kernen, wie N,N-Bis-(2-hydroxyethyl)-anilin und p,p'-Bis-(2-hydroxyethylamino)-diphenylmethan, herstellen. Man kann sie ferner aus einkernigen Phenolen, wie Resorcin und Hydrochinon, und mehrkernigen Phenolen, wie Bis-(4-hydroxyphenyl)-methan (Bisphenol F), 4,4'-Dihydroxybiphenyl, 1,1,2,2-Tetrakis-(4-hydroxyphenyl)-ethan, 2,2-Bis-(4-hydroxyphenyl)-propan (Bisphenol A) und aus durch Umsetzung von Aldehyden, wie Formaldehyd, Acetaldehyd und Benzaldehyd, mit Phenol oder alkylsubstituiertem Phenol, wobei die Alkylgruppen jeweils bis zu neun Kohlenstoffatomen enthalten können, wie 2-Methylphenol und 4-tert.-Butylphenol, gebildeten Novolaken erhalten.

Poly-(N-glycidyl)-verbindungen sind ebenfalls für das erfindungsgemässe Verfahren verwendbar, z.B. N-Glycidylderivate von Aminen, wie Anilin, n-Butylamin, Bis-(4-aminophenyl)-methan und Bis-(4-methylaminophenyl)-methan, Triglycidylisocyanurat sowie N,N'-Diglycidylderivate von cyclischen Alkylenharnstoffen, wie Ethylenharnstoff und 1,3-Propylenharnstoff, und von Hydantoinen, wie 5,5-Dimethylhydantoin.

Man kann auch Poly-(S-glycidyl)-verbindungen einsetzen, z.B. Di-(S-glycidyl)-derivate von Dithiolen, wie Ethan-1,2-dithiol und Bis-(4-mercaptomethylphenyl)-ether, doch sind diese nicht bevorzugt.

Es können auch Glycidylverbindungen, welche zur Flammfestmachung Halogenatome enthalten, wie z.B. Tetrabrombisphenol-A-diglycidylether, verwendet werden.

In Betracht kommen auch Glycidylverbindungen, in welchen die Glycidylgruppen an verschiedene Heteroatome gebunden sind, beispielsweise p-(Diglycidylamino)-phenylglycidylether.

Vorzugsweise setzt man beim erfindungsgemässen Verfahren die Glycidylether, insbesondere die der mehrkernigen Phenole, ein.

Geeignete halogenfreie aprotische organische Lösungsmittel, die beim erfindungsgemässen Verfahren eingesetzt werden können, sind all jene, in welchen das Epoxidharz löslich ist, wie beispielsweise aliphatische Kohlenwasserstoffe, wie Pentan, Hexan, Heptan oder Octan, cycloaliphatische Kohlenwasserstoffe, wie Cyclohexan oder Cyclopentan, aromatische Kohlenwasserstoffe, wie Benzol, Toluol oder Xylole, aliphatische oder cyclische Ether, wie Diethylether, Dioxan oder Tetrahydrofuran, und Ketone, wie Methylethylketon oder Methylisobutylketon, Amide, wie Dimethylformamid, Dimethylacetamid oder Hexamethyl-phosphorsäure-triamid, Nitrile, wie Acetonitril, Propionitril oder Butyronitril, Nitroverbindungen, wie Nitromethan oder Nitrobenzol, Sulfone, wie Tetramethylensulfon, Sulfoxide, wie Dimethylsulfoxid, 1-Methyl-2-pyrrolidon usw.

Vorzugsweise werden als Lösungsmittel Toluol, Methyl-isobutyl-keton, Dimethylsulfoxid, Propionitril, Butyronitril und insbesondere Acetonitril verwendet.

Die im erfindungsgemässen Verfahren einzusetzenden Ammonium-bicarbonate und Alkylcarbonate sind bekannt oder können auf bekannte Weise hergestellt werden. Viele der entsprechenden Ammonium-hydroxide oder -halogenide sind im Handel erhältlich, und die Bicarbonate können daraus durch Umsetzung mit $CO_2$ oder mit einem Alkalimetall-bicarbonat hergestellt werden, wie dies zum Beispiel in der US-Patentschrift 2,873,282 oder in der DE-Offenlegungsschrift 2 723 070 beschrieben ist. Die Ammonium-alkylcarbonate können z.B. durch Umsetzung der entsprechenden Ammoniumalkoxide mit $CO_2$ hergestellt werden. Ammoniumalkoxide sind bekannt oder können auf bekannte Weise hergestellt werden. Sie sind auch im Handel erhältlich. Die Ammoniumhydroxide und Alkoxide können z.B. wie in Analytical Chemistry 28, 787 (1956) beschrieben oder auf analoge Weise hergestellt werden.

Vorzugsweise wird im erfindungsgemässen Verfahren die Glycidylverbindung mit einem Ammonium-bicarbonat der Formel I umgesetzt. Die $C_1$-$C_8$-Alkyl- bzw. $C_1$-$C_8$-Alkoxy Substituenten $R^1$ bis $R^4$ der Verbindungen der Formel I oder II können geradkettig oder verzweigt sein. Als Alkylcarbonat der Formel II wird vorzugsweise ein Ethyl- und insbesondere Methylcarbonat eingesetzt.

Im erfindungsgemässen Verfahren werden vorzugsweise Ammoniumsalze eingesetzt, worin die Substituenten $R^1$ bis $R^4$ unabhängig voneinander Phenyl, Benzyl, $C_1$-$C_4$-Alkyl, insbesondere Methyl sind. Die Substituenten $R^1$ bis $R^4$, können jeweils gleich oder verschieden sein. Bevorzugt werden Ammoniumsalze eingesetzt, bei denen die vier Substituenten am Stickstoffatom jeweils gleich sind. Vorzugsweise können als Ammoniumsalze z.B. Tetraethylammonium-, Tetrapropylammonium-, Phenyltrimethylammonium-, Methyltributylammonium und insbesondere Tetramethylammonium-bicarbonat verwendet werden.

Das im erfindungsgemässen Verfahren entstehende Wasser kann geeigneterweise im Laufe der Umsetzung kontinuierlich entfernt werden, z.B. mit einem Trockenmittel oder insbesondere durch Azeotropdestillation. Geeignete Trockenmittel sind z.B. Aluminiumoxid, Calciumsulfat, Kaliumcarbonat, Magnesiumsulfat, Natriumsulfat, Molekularsiebe, usw. Dem Fachmann sind je nach den Reaktionsbedingungen Lösungsmittel, Temperatur usw. geeignete Trockenmittel bekannt.

Das erfindungsgemässe Verfahren wird vorzugsweise bei einer Temperatur von 10 bis 120°C, insbesondere von 20 bis 100°C durchgeführt. Besonders bevorzugt wird die Umsetzung unter Rückfluss, bei der Siedetemperatur des verwendeten Lösungsmittels durchgeführt. Falls das Lösungsmittel mit dem während der Umsetzung entstehenden Wasser ein Azeotrop bildet, kann das Reaktionswasser dabei unter Rückfluss kontinuierlich aus dem Reaktionsgemisch entfernt werden. Je nach Wunsch kann das erfindungsgemässe Verfahren bei Normaldruck oder bei leicht reduziertem bzw. erhöhtem Druck durchgeführt werden. Vorzugsweise wird bei Normaldruck gearbeitet. Die Reaktionsdauer beträgt im allgemeinen je nach Reaktionsbedingungen 10 min bis 10 Stunden, in den meisten Fällen 1 bis 5 Stunden.

Im erfindungsgemässen Verfahren wird das Ammoniumsalz der Formel I oder II geeigneterweise in einer Menge von 0,1-10 Gew. %, vorzugsweise 0,2 - 5 Gew. %, insbesondere 0,4 - 3,5 Gew. %, ganz besonders bevorzugt 0,5 - 1,5 Gew. %, bezogen auf die Glycidylverbindung, eingesetzt. Dabei kann das Ammoniumsalz entweder als solches dem Reaktionsgemisch beigegeben oder in situ gebildet werden, beispielsweise aus einem entsprechenden quaternären Ammoniumhydroxid und $CO_2$, falls die Reaktion in einer $CO_2$-Atmosphäre durchgeführt wird. Das Ammoniumalkylcarbonat kann auch in situ aus einem entsprechenden quaternären Ammoniumalkoxid und $CO_2$ gebildet werden. Solche quaternären Ammoniumsalze sind dem Fachmann bekannt und im Handel, beispielsweise bei der Fluka AG (Buchs, Schweiz) unter dem Namen Aliquat® (Tricaprylmethylammoniumchlorid), Triton®B (Benzyltrimethylammoniumhydroxid), Hyamine®1622 (Diisobutylphenoxyethoxyethyl-dimethylbenzyl-ammoniumchlorid Monohydrat) oder Hyamine®10-X (Diisobutylcresoxyethoxyethyl-dimethylbenzyl-ammoniumchlorid Monohydrat) erhältlich. Bevorzugt wird im erfindungsgemässen Verfahren das Ammoniumbicarbonat als solches eingesetzt. Das nach

der Reaktion überschüssige Ammoniumsalz und das während der Umsetzung entstandene Ammonium-chlorid werden aus dem Reaktionsgemisch geeigneterweise durch Filtration, Adsorption oder insbesondere durch Waschen mit Wasser entfernt. Dabei kann als Adsorbens z.B. Kieselgel verwendet werden. Nach dem Entfernen der Ammoniumsalze aus dem Reaktionsgemisch wird das restliche organische Lösungsmittel geeigneterweise abdestilliert und die Glycidylverbindung mit vermindertem Chlorgehalt isoliert.

Die vorliegende Erfindung betrifft auch die nach dem erfindungsgemässen Verfahren erhältlichen Glycidylverbindungen mit vermindertem Chlorgehalt. Wegen ihres tiefen Chlorgehaltes eignen sich die erfindungsgemäss erhaltenen Glycidylverbindungen insbesondere für Anwendungen, bei denen sich die Korrosion der mit den Harzen in Kontakt stehenden Metalle störend auswirkt, wie z.B. bei der Elektrobeschichtung von Metallen, wie Elektrotauchlackierung, Kataphorese usw., und insbesondere für Anwendungen in der Elektrotechnik und Elektronik.

Die Harze können in jeweils dem speziellen Anwendungszweck angepasster Formulierung, in ungefülltem oder gefülltem Zustand, z.B. als Komponente von Pressmassen, Wirbelsinterpulvern, Giessharzen, Spritzgussformulierungen, Imprägnierharzen, Klebmitteln, Werkzeugharzen, Laminierharzen und Pulverlacken eingesetzt werden.

Insbesondere eignen sich die erfindungsgemäss erhaltenen Glycidylverbindungen als Umhüllungsharze für elektronische Komponenten, als Klebstoffe oder Schutzüberzüge bei der Befestigung elektronischer Komponenten auf Leiterplatten ("globe top", "conformer coating").

Die in den folgenden Beispielen angegebenen Mengen an nicht hydrolysierbarem und an hydrolysierbarem Chlor werden wie folgt bestimmt:

Gesamtchlorgehalt: Zu etwa 3 g der Glycidylverbindung werden 25 ml Butylcarbitol (Diethylenglykolmonobutylether) und 25 ml einer 1N KOH-Lösung in 1,2-Propandiol zugegeben. Die Lösung wird 10 Minuten

unter Rückfluss erhitzt und anschliessend auf Zimmertemperatur abgekühlt. Nach Zugabe von 50 ml Essigsäure wird der Chlorgehalt mit einer 0,01 N $AgNO_3$-Lösung bestimmt.

Gehalt an hydrolysierbarem Chlor: Etwa 5 g der Glycidylverbindung werden in 20 ml Toluol gelöst und mit 50 ml einer 0,1 N KOH-Lösung in Methanol versehen. Die Lösung wird 2 Minuten unter Rückfluss erhitzt und dann auf 10-15°C gekühlt. Nach Zugabe von 50 ml Essigsäure wird der Chlorgehalt mit einer 0,01 N $AgNO_3$-Lösung bestimmt.

Der Gehalt an nichthydrolysierbarem Chlor wird durch Subtraktion des Gehaltes an hydrolysierbarem Chlor vom Gesamtchlorgehalt bestimmt.

In den folgenden Beispielen wird der Chlorgehalt der Epoxidharze in ppm, bezogen auf das Gesamtgewicht des Harzes, angegeben.

Beispiel 1: 100 g eines aus Bisphenol-A und Epichlorhydrin hergestellten Bisphenol-A-diglycidylethers mit einem Epoxidgehalt von 5,36 Aequivalenten/kg, einem Gehalt an nichthydrolysierbarem Chlor von 1295 ppm und einem Gehalt an hydrolysierbarem Chlor von 127 ppm werden in 100 g Toluol gelöst, und 3 g Tetramethylammonium-bicarbonat werden zugegeben. Das Gemisch wird in einer $CO_2$-Atmosphäre unter Rückfluss 75 min erhitzt, und das während der Umsetzung entstehende Wasser wird als Azeotrop entfernt. Nach Beendigung der Reaktion wird das Gemisch mit Wasser gewaschen, wodurch das bei der Reaktion entstandene Tetramethylammonium-chlorid sowie das überschüssige Tetramethylammoniumbicarbonat entfernt werden. Der nach dem Abdestillieren des Toluols erhaltene Bisphenol-A-diglycidylether weist einen Epoxidgehalt von 4,53 Aequivalenten/kg, einen Gehalt an nichthydrolysierbarem Chlor von 559 ppm und einen Gehalt an hydrolysierbarem Chlor von 50 ppm auf.

Beispiel 2: Beispiel 1 wird wiederholt, indem statt Toluol Methylisobutyl-keton als Lösungsmittel verwendet wird. Der so erhaltene Bisphenol-A-diglycidylether weist einen Epoxidgehalt von 4,45 Aequivalenten/kg, einen Gehalt an nichthydrolysierbarem Chlor von 581 ppm und einen Gehalt an hydrolysierbarem Chlor von 60 ppm auf.

Beispiel 3:: 200 g des im Beispiel 1 eingesetzten Epoxidharzes werden in 200 g Toluol gelöst, 3 g Tetramethylammoniumhydroxid werden beigegeben, und das Gemisch wird während 30 min bei Raumtemperatur gerührt und dann unter $CO_2$-Atmosphäre während 4 Stunden bei 50°C erhitzt. Nach Beendigung der Reaktion wird das Reaktionsgemisch mit Wasser gewaschen, und das Toluol wird abdestilliert. Der erhaltene Bisphenol-A-diglycidylether weist einen Epoxidgehalt von 4,93 Aquivalenten/kg, einen Gehalt an nichthydrolysierbarem Chlor von 749 ppm und einen Gehalt an hydrolysierbarem Chlor von 94 ppm auf.

Beispiel 4: 200 g eines Epoxykresolnovolaks mit einem Epoxidgehalt von 4,62 Aequivalenten/kg, einem Gehalt an nichthydrolysierbarem Chlor von 1300 ppm und einem Gehalt an hydrolysierbarem Chlor von 28 ppm werden in 200 g Acetonitril gelöst, und 5 g Tetramethylammoniumbicarbonat werden beigegeben. Das Gemisch wird in einer $CO_2$-Atmosphäre während 4 Stunden unter Rückfluss erhitzt, und das entstehende Wasser wird als Azeotrop entfernt. Nach der Umsetzung wird das Gemisch mit Wasser gewaschen, und das restliche Acetonitril wird durch Destillation entfernt. Das erhaltene Harz weist einen Epoxidgehalt von 4,24 Aequivalenten/kg, einen Gehalt an nichthydrolysierbarem Chlor von 873 ppm und an hydrolysierbarem Chlor von 56 ppm auf.

Beispiel 5: 100 g eines flüssigen Bisphenol-A-diglycidylethers mit einem Epoxidgehalt von 5,32 Aquivalenten/kg, einem Gehalt an nichthydrolysierbarem Chlor von 1315 ppm und einem Gehalt an hydrolysierbarem Chlor von 127 ppm werden in 100 g Acetonitril gelöst und mit 2,5 g Tetramethylammonium-bicarbonat unter $CO_2$ während 4 Stunden unter Rückfluss erhitzt, wobei das entstehende Wasser kontinuierlich

als Azeotrop entfernt wird. Die abgekühlte Lösung wird dreimal mit Wasser gewaschen, und das restliche Acetonitril wird durch Destillation entfernt. Das erhaltene Epoxidharz weist einen Epoxidgehalt von 5,16 Aequivalenten/kg, einen Gehalt an nichthydrolysierbarem Chlor von 841 ppm und einen Gehalt an hydrolysierbarem Chlor von 38 ppm auf.

Beispiel 6: Beispiel 5 wird mit einem Bisphenol-A-diglycidylether mit einem Epoxidgehalt von 5,32 Aquivalenten/kg, einem Gehalt an nichthydrolysierbarem Chlor von 910 ppm und an hydrolysierbarem Chlor von 90 ppm wiederholt. Nach der Umsetzung weist der Bisphenol-A-diglycidylether einen Epoxidgehalt von 5,07 Aequivalenten/kg, einen Gehalt an nichthydrolysierbarem Chlor von 503 ppm und an hydrolysierbarem Chlor von 30 ppm auf.

Beispiel 7: 100 g des im Beispiel 1 eingesetzten Epoxidharzes werden mit 0,6 g Tetramethylammoniumbicarbonat in 100 g Acetonitril während 5 Stunden unter Stickstoff rückflussiert. Die Lösung wird durch Kieselgel (Kieselgel 60, 70-230 mesh, Merck, Nr. 7734) filtriert und nach Entfernung des Lösungsmittels wird ein Harz mit einem Epoxidgehalt von 5,26 Aequivalenten/kg, einem Gesamtchlorgehalt von 746 ppm, einem Gehalt an hydrolysierbarem Chlor von 23 ppm und an ionischem Chlor von weniger als 1 ppm erhalten.

Beispiel 8: 100 g des im Beispiel 1 eingesetzten Epoxidharzes werden mit 1 g Benzyltrimethylammonium-methylcarbonat (hergestellt in situ aus Benzyltrimethylammonium-methoxid und $CO_2$) in 100 g Acetonitril unter $CO_2$ während 2 Stunden rückflussiert. Die Lösung wird durch Kieselgel filtriert und das Lösungsmittel entfernt. Das Epoxidharz hat einen Gesamtchlorgehalt von 991 ppm, einen Gehalt an hydrolysierbarem Chlor von 40 ppm und an ionischem Chlor von weniger als 1 ppm.

Beispiel 9: 100 g eines Epoxykresolnovolaks mit einem Epoxidgehalt von 5,12 Aequivalenten/kg, einem Gesamtchlorgehalt von 1271 ppm, einem Gehalt an hydrolysierbarem Chlor von 14 ppm und an ionischem

Chlor von weniger als 1 ppm werden mit 0,6 g Tetramethylammoniumbicarbonat während einer halben Stunde unter Stickstoff in 100 g Propionitril rückflussiert, und das Reaktionsgemisch wird durch Kieselgel filtriert. Nach Entfernung des Lösungsmittels erhält man ein Harz mit einem Epoxidgehalt von 5,06 Aequivalenten/kg, einem Gesamtchlorgehalt von 1014 ppm, einem Gehalt an hydrolysierbarem Chlor von 72 ppm und an ionischem Chlor von weniger als 1 ppm.

<u>Beispiel 10:</u> Beispiel 9 wird unter Verwendung von Butyronitril als Lösungsmittel wiederholt. Das erhaltene Epoxidharz hat einen Epoxidgehalt von 4,82 Aequivalenten/kg, einen Gesamtchlorgehalt von 742 ppm, einen Gehalt an hydrolysierbarem Chlor von 104 ppm und an ionischem Chlor von weniger als 1 ppm.

<u>Beispiele 11 bis 14:</u> 100 g des im Beispiel 1 eingesetzten Epoxidharzes werden mit der in der Tabelle angegebenen Menge des Ammoniumsalzes jeweils in 100 g Acetonitril unter $CO_2$ während zwei Stunden rückflussiert. Nach Aufarbeitung des Reaktionsgemisches (Filtration durch Kieselgel und Entfernung des Lösungsmittels) werden die in der Tabelle spezifizierten Harze erhalten.

Tabelle

| Bsp. | $R^4-\overset{R^1}{\underset{R^3}{N^+}}-R^2\ HCO_3^-$ | Gesamt-Cl (ppm) | Produkt hydrolysierbares Cl (ppm) | ionisches Cl (ppm) | Epoxid-äquivalent/kg |
|---|---|---|---|---|---|
| 11 | $R^1-R^4$ =n.Propyl 1,09 g | 958 | 48 | < 1 | 5,06 |
| 12 | $R^1-R^3$ =n-Butyl 1,15 g<br>$R^4$ =Methyl | 932 | 30 | < 1 | 5,06 |
| 13 | $R^1-R^3$ =Methyl 0,88 g<br>$R^4$ =Phenyl | 993 | 35 | < 1 | 5,20 |
| 14 | $R^1-R^4$ =Ethyl 0,84 g | 973 | 31 | < 1 | 5,16 |

Beispiel 15: 100 g eines Bisphenol-F-diglycidylethers mit einem Epoxidgehalt von 6,20 Aequivalenten/kg, einem Gesamtchlorgehalt von 1100 ppm, einem Gehalt an hydrolysierbarem Chlor von 2 ppm und an ionischem Chlor von weniger als 1 ppm werden mit 0,6 g Tetramethylammoniumbicarbonat in 100 g DMSO unter $CO_2$ bei 80°C während 2 Stunden erhitzt. Das Reaktionsgemisch wird mit Wasser gewaschen und das Lösungsmittel wird entfernt. Das so erhaltene Harz hat einen Epoxidgehalt von 5,71 Aequivalenten/kg, einen Gesamtchlorgehalt von 248 ppm, einen Gehalt an hydrolysierbarem Chlor von 18 ppm und an ionischem Chlor von weniger als 1 ppm.

Patentansprüche

1. Verfahren zur Verminderung des Chlorgehaltes in Glycidylverbindungen, deren Glycidylgruppen an O-, N- und/oder S-Atome gebunden sind, indem man die in einem halogenfreien aprotischen organischen Lösungsmittel gelöste Glycidylverbindung mit einem Ammonium-bicarbonat oder -alkylcarbonat reagieren lässt, und danach das Produkt aus der Reaktionslösung isoliert, dadurch gekennzeichnet, dass das Ammoniumsalz eine Verbindung der Formeln I oder II

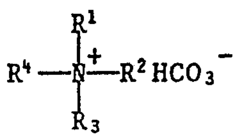

I

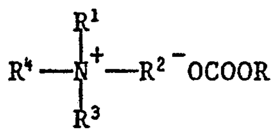

II

ist, worin $R^1$ bis $R^4$ unabhängig voneinander $C_1$-$C_8$-Alkyl, Phenyl, Benzyl, $C_1$-$C_8$-Alkoxy oder Phenoxy und R $C_1$-$C_4$-Alkyl bedeuten.

2. Verfahren nach Anspruch 1, worin die Glycidylverbindung ein Glycidylether, insbesondere ein Polyglycidylether eines mehrkernigen Phenols ist.

3. Verfahren nach Anspruch 1, worin man die Glycidylverbindung mit einem Ammonium-bicarbonat der Formel I reagieren lässt.

4. Verfahren nach Anspruch 1, worin die Substituenten $R^1$ bis $R^4$ unabhängig voneinander Phenyl, Benzyl oder $C_1$-$C_4$-Alkyl, insbesondere Methyl sind.

5. Verfahren nach Anspruch 1, worin das Alkylcarbonat ein Ethyl- und insbesondere Methylcarbonat ist.

6. Verfahren nach Anspruch 3, worin das Ammoniumbicarbonat ein Tetraethylammonium-, ein Tetrapropylammonium-, ein Phenyltrimethylammonium-, ein Methyltributylammonium und insbesondere ein Tetramethylammonium-bicarbonat ist.

7. Verfahren nach Anspruch 1, welches unter einer $CO_2$- oder $N_2$-Atmosphäre durchgeführt wird.

8. Verfahren nach Anspruch 1, worin das entstandene Reaktionswasser mit einem Trockenmittel oder insbesondere durch Azeotropdestillation entfernt wird.

9. Verfahren nach Anspruch 1, worin das nach der Reaktion überschüssige Ammonium-bicarbonat und das während der Umsetzung entstandene Ammonium-chlorid durch Filtration, Adsorption oder durch Waschen mit Wasser entfernt werden.

10. Verfahren nach Anspruch 1, worin das Ammonium-bicarbonat als solches eingesetzt wird.

11. Verfahren nach Anspruch 7, worin das Ammonium-bicarbonat in situ aus einem entsprechenden Ammonium-hydroxid und $CO_2$ gebildet wird.

12. Verfahren nach Anspruch 7, worin das Ammoniumalkylcarbonat in situ aus einem entsprechenden quaternären Ammoniumalkoxid und $CO_2$ gebildet wird.

13. Verfahren nach Anspruch 1, worin 0,1-10 Gew. %, vorzugsweise 0,2-5 Gew. % des Ammoniumsalzes, bezogen auf die Glycidylverbindung, eingesetzt werden.

14. Verfahren nach Anspruch 1, dadurch gekennzeichnet, dass man die Reaktion im Temperaturbereich von 10 bis 120, vorzugsweise von 20-100°C, durchführt.

15. Verwendung der Glycidylverbindungen hergestellt nach dem Verfahren nach Anspruch 1 als Umhüllungsharze für elektronische Komponenten, als Klebstoffe oder Schutzüberzüge bei der Befestigung elektronischer Komponenten auf Leiterplatten ("globe top", "conformer coating").

FO 7.3 SZ/cs*